(19) Europäisches Patentamt
European Patent Office
Office européen des brevets

(11) Publication number: 0 049 354
A2

# (12) EUROPEAN PATENT APPLICATION

(21) Application number: 81106483.1

(22) Date of filing: 20.08.81

(51) Int. Cl.[3]: A 61 K 31/19
A 61 K 9/70

(30) Priority: 08.09.80 US 185149

(43) Date of publication of application:
14.04.82 Bulletin 82/15

(84) Designated Contracting States:
BE CH DE FR GB IT LI NL SE

(71) Applicant: STERLING DRUG INC.
90 Park Avenue
New York New York(US)

(72) Inventor: Diana, Guy Dominic
Box 192 Garfield Road
East Nassau New York(US)

(74) Representative: Baillie, Iain Cameron et al,
c/o Ladas & Parry Isartorplatz 5
D-8000 München 2(DE)

## (54) Composition for therapeutic virucidal use, and articles and packages containing said composition.

(57) The compositions, articles and packages for the therapeutic use in neutralizing or destroying viruses with glutaric acid are disclosed.

EP 0 049 354 A2

The invention relates to glutaric acid containing compositions, articles and packages for therapeutic antiviral use.

It has been shown (Hendley, Wenzel and Gwaltney, New England Journal of Medicine, vol. 288, pp. 1361-1364, 1973) that colds caused by rhinoviruses can be transmitted from person to person or from person to object to person by way of the hands. It is believed that self-infection takes place by transfer of virus from the fingers to the nasal mucosa or the conjunctiva. Similar transmission of other types of virus is also believed possible. The need for a virucidal compositions, especially for the hands, is satisfied by the invention. A most special need satisfied by the invention is for compositions reducing rhinovirus titers in the respiratory tracts of mammals infected by rhinoviruses and thereby reducing the severity, discomfort and contagiousness of such infections.

Gluratic acid is a well-known organic compound having the structural formula

$$HOOCCH_2CH_2CH_2COOH$$

which is not known for its pharmaceutical uses, microbiocidal or other. In fact, the literature (Gershon and Shanks, Canadian Journal of Microbiology, vol. 22, no. 8, pp. 1198-1201, 1976; Chemical Abstracts, vol. 85, 117344m, 1976) describes the antifungal testing of the alkanedioic acids of 2-12, 14 and 16 carbon atoms and the dimethyl diesters thereof and states that "[t]he dicarboxylic acids possessed very poor to no antifungal activity against all 6 fungi"

(Asperigillus niger, Trichoderma veride, Myrothecium verrucaria, Candida albicans, Trichophyton mentagrophytes, Mucor mucedo).

In a test against Sarcoma 180 tumor in mice glutaric acid was found to have no gradeable effect on the tumor (Stock et al., Cancer Research, vol. 20, pp. 193-195 and 236, 1960). The test was done by injection with a formulation of glutaric acid in a carboxymethylcellulose (0.5%)-saline vehicle.

The property of glutaric acid as a therapeutically active virucidal substance has been heretofore unknown. The invention reflects the discovery of this property.

One aspect of the invention is a composition for neutralizing or destroying a susceptible virus on infected tissue of a living mammal comprising a virucidally effective concentration of glutaric acid and a pharmaceutically acceptable carrier.

Another aspect of the invention is a paper or cloth article coated or impregnated with a virucidally effective amount of glutaric acid alone or a composition consisting essentially of a virucidally effective amount of gluratic acid and a pharmaceutically acceptable carrier.

A further aspect of the invention is a package which consists of glutaric acid alone, a composition consisting essentially of virucidally effective concentration of glutaric acid and a pharmaceutical vehicle, or a paper or cloth article coated or impregnated with a virucidally effective amount of glutaric acid alone or a composition consisting essentially of a virucidally effective amount of glutaric acid and a pharmaceutically acceptable vehicle, and a container with indicia for virucidal use of the glutaric acid, the composition or the article.

Virucidal is defined (Dorland's Illustrated Medical Dictionary, Twenty-fifth Edition, 1974) as "capable of neutralizing or destroying a virus". A susceptible virus is considered to mean one which is neutralized or destroyed by glutaric acid. The susceptible viruses are readily

identified in tests such as those described below, wherein the amount or concentration of glutaric acid is considered virucidal if the virus titer is reduced by 99.9% or more.

Examples of suceptible viruses are rhinovirus, herpes virus, influenza virus, para-influenza virus and respiratory syncytial virus.

Test of Activity of Glutaric Acid Intranasally Against Rhinovirus Infection in Hamsters

Equine rhinovirus (TC pool X-10 prepared in Vero cells, titer-log $_{10}$=8.0) instilled into the nostrils (0.05 ml./nostril) of hamsters, persisted in their nasal passages for at least 7 days. Periodic washing of the nasal passages during the post-infection period showed appreciable virus titers on days 3 and 4 and lower titers on day 7. Virus titers in nasal washes on days 3 and 4 postinfection were similar to those reported in human rhinovirus infections and ranged from 2.0 to 3.0 log $TCID_{50}$/0.2 ml. Because of the physical and chemical similarity of equine rhinovirus to human rhinovirus and because it will infect laboratory animals, equine rhinovirus was used in the hamster model to study the effect of glutaric acid applied intranasally. Treatment of the infected hamsters with glutaric acid as 2%, 5% or 10% solutions, administered intranasally either with a Teflon tipped syringe as droplets containing 0.45% lactic acid and adjusted to pH 3.5 or as an aerosol spray adjusted to pH 4.0, resulted in a significant reduction (>90%) of virus titer on each sampling day (days 3,4 and 7) by the two highest concentrations (5% and 10%) when compared to placebo controls.

Test of Activity of Glutaric Acid Against Herpesvirus Hominis Type 2 in Mouse Genital Infection

Intravaginal administration of aqueous solutions of glutaric acid at concentrations of 1, 2, 4, 8 and 10% to mice infected by the intravaginal route with herpesvirus hominis type 2 resulted in increased survival rate of the mice compared with placebo-treated controls as shown by the

following table.

| Glutaric Acid Concentration (%) | Survival Rate (%) |
|---|---|
| Placebo | 10 |
| 1 | 30 |
| 2 | 60 |
| 4 | 50 |
| 8 | 60 |
| 10 | 70 |

The compositions of the invention are intended for therapeutic virucidal use. For this purpose the glutaric acid can be formulated in any appropriate pharmaceutically acceptable carrier provided that the glutaric acid and the carrier are compatible, that is, that the virucidal activity of the glutaric acid is not diminished by the carrier. The compositions can be in the form of creams, foams, lotions, ointments, solutions or sprays. The carrier diluent can be aqueous or non-aqueous, for example alcoholic or oleaginous, or a mixture thereof, and may additionally contain surfactants, emollients, lubricants, stabilizers, dyes, perfumes, antimicrobial agents either as active ingredients or as preservatives and acid or base for adjustment of pH. The preferred pH is about 4. Conventional methods are used in preparing the compositions.

The foregoing compositions can be dispensed in pre-moistened pads or tissues. The latter can be packaged individually as described, for example, in U.S. Pat. 3,057,467 or multiply, in separate sheets as described, for example, in U.S. Pat. 3,836,044 or in a roll as described, for example, in U.S. Pat. 4,017,002.

The paper or cloth articles of the invention are coated or impregnated with glutaric acid or a glutaric acid composition by dipping, spreading, spraying, dusting or condensing glutaric acid vapor and are otherwise made by known methods of manufacture.

Dry facial tissues containing glutaric acid at concentrations of 0.5, 1.0 and 2.0 mg. per square inch, which

were prepared by spraying aqueous solutions of glutaric acid onto commercially available paper tissues and drying them, inactivated rhinovirus type 2 by greater than 99.99% after a 10 min. contact at room temperature.

The package aspect of the invention combines the other aspects of the invention and is the form in which the invention is contemplated to be sold. The container can be of any type for solid or liquid contents. The indicia for therapeutic virucidal use of the composition or article of the invention can be a label, package insert or any other means of communicating such use.

Example 1

The following example is a composition intended for use as a virucidal lotion wherein the vehicle is an aqueous surfactant-emollient mixture.

| Ingredient | Percent by Weight |
|---|---|
| Glutaric Acid | 1.00xxx |
| Glyceryl Stearate (and) PEG-100 Stearate | 3.00xxx |
| PPG-12-Buteth-16 | 2.00xxx |
| Cetyl Alcohol | 1.50xxx |
| Myristyl Alcohol | 1.50xxx |
| PEG-4 Laurate | 1.00xxx |
| PEG-4 Stearate | 0.500xx |
| Glycerin | 0.500xx |
| Perfume | 0.300xx |
| Carbomer-934P | 0.200xx |
| Zinc Pyrithione | 0.100xx |
| Dye | 0.00200 |
| Water to make | 100.00xxx |

Example 2

The composition corresponding to the composition of Example I except that 2.00% glutaric acid was substituted for 1.00% glutaric acid was also prepared.

Example 3

The following example is a composition intended for use as a virucidal foam wherein the vehicle is an aqueous ethyl alcoholic surfactant-emollient aerosol mixture.

| Ingredient | Percent by Weight |
| --- | --- |
| Glutaric Acid | 1.00xx |
| Steareth-2 | 1.50xx |
| Laneth-16 | 1.00xx |
| Cetyl Alcohol | 0.800x |
| Myristyl Alcohol | 0.200x |
| Perfume | 0.0500 |
| Alcohol, USP | 54.2xxx |
| Purified Water | 36.3xxx |
| Propane | 0.800x |
| Isobutane | 4.20xx |
| Water to Make | 100.0xxx |

Example 4

A composition corresponding to the composition of Example 3 except that 2.00% glutaric acid was substituted for 1.00% glutaric acid was also prepared.

CLAIMS

1. A composition for therapeutic virucidal use characterized by a virucidally effective concentration of glutaric acid and a pharmaceutically acceptable carrier.

2. A composition according to claim 1, characterized in that the carrier is an aqueous surfactant emollient mixture.

3. A composition according to claim 1, characterized in that the carrier is an aerosol mixture.

4. A composition according to claim 1, characterized by being incorporated in pre-moistened pads or tissues.

5. A composition according to any one of claims 1 to 4, characterized by additionally containing an antimicrobially effective amount of an antimicrobial agent.

6. A composition according to claim 5, characterized in that the antimicrobial agent is sodium benzoate, dehydroacetic acid, hexyl-resorcinol or phenoxyethanol.

7. A paper or cloth article for therapeutic virucidal use, characterized by being coated or impregnated with a virucidally effective amount of glutaric acid alone or a composition consisting essentially of a virucidally effective concentration of glutaric acid and a pharmaceutically acceptable carrier.

8. An article according to claim 7, characterized by being a dry facial tissue.

9. A package for therapeutic virucidal use, characterized by consisting glutaric acid alone, a composition consisting essentially of a virucidally effective concentration of glutaric acid and a pharmaceutical carrier or a paper or cloth article coated or impregnated with a virucidally effective amount of glutaric acid alone or a composition consisting essentially of a virucidally effective concentration of glutaric acid and a pharmaceutically acceptable carrier and a container with indicia for rhinovirucidal use of the glutaric acid, the composition or the article.